# EUROPEAN PATENT SPECIFICATION

(11) **EP 1 066 043 B1**
(45) Date of publication and mention of the grant of the patent: **17.12.2003**
(21) Application number: 99913481.0
(22) Date of filing: 29.03.1999
(51) Int. Cl.: A61K 31/7004, A61K 39/395, A61P 37/08

(54) **MATERIALS FOR THE TREATMENT OF CONDITIONS INVOLVING MAST CELLS, BASOPHILS AND EOSINOPHILS**
MATERIALIEN ZUR BEHANDLUNG VON ZUSTÄNDEN, BEI DENEN BASOPHILE- ODER EOSINOPHILE-GRANULOZYTEN ODER MASTZELLEN EINE ROLLE SPIELEN
MATIERES POUR LE TRAITEMENT DE TROUBLES IMPLIQUANT DES MASTOCYTES, DES BASOPHILES ET DES EOSINOPHILES

(30) Priority: 27.03.1998 GB 9806645
(43) Date of publication of application: 10.01.2001
(73) Proprietor: UNIVERSITY COLLEGE LONDON, London WC1E 6BT (GB)
(72) Inventor: RADEMACHER, Thomas William, Boars Hill, Oxford OX1 5EY (GB); WHITBY, Helen, London W1N 1AD (GB)
(74) Representative: Kiddle, Simon John
(86) International application number: GB9900981
(87) International publication number: WO99049855

(56) References cited:
- WO-A-96/29425
- WO-A-98/10791
- WO-A-98/11116
- WO-A-98/11117
- WO-A-98/11435
- RADEMACHER T W ET AL: "Inositolphosphoglycan second messengers." BRAZILIAN JOURNAL OF MEDICAL AND BIOLOGICAL RESEARCH, (1994 FEB) 27 (2) 327-41. , XP002050248 cited in the application

## Description

### Field of the Invention

The present invention relates to materials and methods relating to the treatment of conditions involving mast cells, basophils and eosinophils, and in particular to inositolphosphoglycans (IPGs) as obtainable from mast cells, basophils or eosinophils, and to uses of inositolphosphoglycan (IPG) antagonists in the treatment of conditions that are mediated by the release of IPGs from mast cells, basophils or eosinophils, such as allergies or asthma.

### Background of the Invention

Allergy affects twenty percent of the worlds' population and the alarming increase in its prevalence, morbidity and mortality over the past decade has led to its designation as the number one environmental disease (Sutton and Gould, 1993). Scientists have become increasingly interested in the mechanisms of allergy and the potential benefits of discovering therapeutics to block these mechanisms.

The traditional model of type one hypersensitivity (acute allergic reaction), involves the cross-linking of IgE receptors on basophils and mast cells by the antigen. The cross-linking leads to receptor clustering, degranulation of vesicles and the release of pre-formed mediators, such as histamine. In addition, newly formed mediators such as prostaglandins and leukotrienes are generated (Sampson et al, 1989). This reaction is fast and relatively easy to resolve with anti-histamines and steroids.

In certain individuals, a more malignant form of allergy is seen after the acute allergic reaction subsides, which is characterised by a recrudescence of symptoms after a period of 3 to 11 hours. This type of reaction, the Late Phase Reaction (LPR), occurs in chronic allergic diseases such as chronic asthma and eczema (Kuna et al, 1993). LPR in the skin is characterised by oedema and erythema (Solley et al, 1976), and in the nose by increased resistance of airflow (MacLean et al, 1971). The pathogenesis of the LPR is complex, poorly understood and difficult to resolve with currently available standard therapies.

Research into the pathogenesis of LPR, revealed an eight fold increase in basophil numbers and a marked absence of the classic mast cell marker, prostaglandin 2. It was postulated, therefore, that the cell of significance in the LPR was the basophil (Lichtenstein, 1988). During experiments to ascertain the influence of the immune system on the function of basophils, it was determined that cultured macrophages produced a factor which effected histamine release from human basophils (Liu et al, 1986) in the absence of antigen persistence (Lichtenstein, 1988). The experiment was repeated using a factor found in fluids derived from the LPR skin blisters (MacDonald et al, 1995) and with nasal washings from both atopic and non-atopic individuals (Sim et al, 1992), and the factor was designated histamine releasing factor (HRF) .

Subsequent experimental studies found that study groups, consisting of approximately 50% of atopic individuals, reacted to HRF, although it should be noted that the percentage varies depending on the nature of the atopic disease (50% in atopic rhinitis compared with 70% in atopic asthma) (Fischer et al, 1987).

Many of the actions of growth factors on cells are thought to be mediated by a family of inositol phosphoglycan (IPG) second messengers (Rademacher et al, 1994). It is thought that the source of IPGs is a "free" form of glycosyl phosphatidylinositol (GPI) situated in cell membranes. IPGs are thought to be released by the action of phosphatidylinositol-specific phospholipases following ligation of growth factors to receptors on the cell surface. There is evidence that IPGs mediate the action of a large number of growth factors including insulin, nerve growth factor, hepatocyte growth factor, insulin-like growth factor I (IGF-I), fibroblast growth factor, transforming growth factor β, the action of IL-2 on B-cells and T-cells, ACTH signalling of adrenocortical cells, FSH and hCG stimulation of granulosa cells, thyrotropin stimulation of thyroid cells, cell proliferation in the early developing ear and rat mammary gland.

Soluble IPG fractions have been obtained from a variety of animal tissues including rat tissues (liver, kidney, muscle brain, adipose, heart) and bovine liver. IPG biological activity has also been detected in malaria parasitized RBC and mycobacteria. We have divided the family of IPG second messengers into distinct A and P-type subfamilies on the basis of their biological activities. In the rat, release of the A- and P-type mediators has been shown to be tissue-specific (Kunjara et al, 1995).

However, until very recently, it was not possible to isolate single purified components from the tissue derived IPG fractions, much less in sufficient quantities to allow structural characterisation. Accordingly, prior art studies were based on the biological activities of the IPG containing fractions, and speculation as to the identity of the active components from non-human sources of the fractions were based on indirect evidence from metabolic labelling and cleavage techniques.

### Summary of the Invention

Broadly, the present invention is based on the finding that IPGs can be obtained from basophils, eosinophils and mast cells and that allergen stimulation of these cells results in IPG release. The experiments also show that IPGs are second messengers for allergic stimulation as the addition of some types of purified IPGs to non-allergen stimulated cells resulted in histamine release or degranulation.

Accordingly, in a first aspect, the present invention provides an inositolphosphoglycan (IPG) as obtainable from mast cells, basophils or eosinophils which is capable of causing histamine release from mast cells, basophils or eosinophils.

In a further aspect, the present invention provides the use of an inositolphosphoglycan (IPG) as obtainable from mast cells, basophils or eosinophils which is capable of causing histamine release from mast cells, basophils or eosinophils in a method of screening for antagonists of said IPG.

In a further aspect, the present invention provides the use of an IPG antagonist in the preparation of a medicament for the treatment of conditions mediated by the release of IPGs from mast cells, basophils or eosinophils wherein the antagonist is a substance as defined in claim 1.

In a further aspect, the present invention provides a method of preventing the release of IPGs from mast cells, basophils or eosinophils, the method comprising exposing the mast cells, basophils or eosinophils to an IPG antagonist.

In a further aspect, the present invention provides a method of treating a condition mediated by release of IPGs from mast cells, basophils or eosinophils, the method comprising administering an effective amount of an IPG antagonist to a patient.

Preferably, the IPG antagonist acts specifically on mast cells, basophils and/or eosinophils. However, the activity of the antagonist can be provided in a number of ways. In one embodiment, the IPG antagonist can be an antibody capable of specifically binding to IPGs, in particular IPGs produced by mast cells, basophils or eosinophils. The use of neutralising antibodies is preferred, e.g. antibodies capable of neutralising one or more of the activities of IPGs on mast cells, basophils or eosinophils, e.g stimulating histamine release.

Alternatively, the IPG antagonists can act to inhibit or prevent IPG release in mast cells, basophils or eosinophils, e.g. in response to an allergen. An example of this type of antagonist is a specific inhibitor of the enzyme GPI-PLD, which is involved in the release of the IPG from the mast cell, basophil or eosinophil surface following allergen stimulation. A further example is an anti-GPI-PLD antibody which acts to inhibit IPG release by inhibiting cleavage of the IPGs caused by the enzyme GPI-PLD.

As the action of IPGs is generally tissue specific, IPGs derived from other tissues can provide a source of competitive antagonists to IPGs released from mast cells, basophils or eosinophils, i.e. substances which compete with the mast cell, basophil or eosinophil derived IPGs, but which do not share their activity in relation to mast cells, basophils or eosinophils, e.g. do not cause histamine release. An example of this type of antagonist is the rat liver A-type IPG described in the examples below, which in combination with an adjunct (in this case Ca²⁺) was antagonised the hexosaminidase release from basophil cell line RBL 2H3. Other IPGs could be screened for mast cell, basophil or eosinophil antagonist activity using the assays described below.

Other suitable IPG antagonists can be prepared and screened by those skilled in the art based on the detailed teaching below.

The present invention is applicable to the treatment of a range of disorders mediated by IPG release from mast cells, basophils or eosinophils. These include conditions mediated by the following mediators and cytokines, the IPGs acting a second messengers for these mediators or cytokines:
(a) Preformed mediators, including histamine, HRF, and neutral proteases.
(b) Newly generated mediators (lipid derived), including prostaglandins (PGD₂,PGF₂α) thromboxanes and leukotrienes.
(c) Cytokines, including IL-3, IL-4, IL-5, IL-6, IL-8 GM-CSF, TNFα, IFNα, MIP 1α/1β, T-cell activation antigen.
(d) Other mediators, including PAF and RANTES.

The conditions that can be prevented or treated using the IPG antagonists include atopic dermatitis, food hypersensitivity, allergies including seasonal, contact, drug, pollen, insect allergies, asthma (both early and late phase), allergic interstitial pneumonitis, eczema, environmental lung disease, and other disorders mediated by infiltration of mast cells, basophils or eosinophils, or cells within their respective lineages.

Embodiments of the present invention will now be described by way of example and not by limitation with reference to the accompanying drawings.

### Brief Description of the Drawings

Figure 1 shows that IPGs obtained from a basophil cell line RBL 2H3 cause the release of histamine from the RBL 2H3 cells. Figure 1 shows % histamine release from the cells plotted against amount of IPG added.
Figure 2 shows that the response of the basophils is tissue-specific since IPGs isolated from human placental were not able to cause histamine release (the hexosaminidase release (y axis) is a surrogate marker for histamine release).
Figure 3 shows that rat liver P-type IPG has no effect on the RBL 2H3 cells. In contrast, the rat A-type liver IPG was able to block some of the spontaneous release. The effect is specific since no inhibition was seen in the absence of calcium (Figure 3 bottom).
Figure 4 shows results from experiments to determine the role of GPI-PLD in Type One hypersensitivity reactions.

### Detailed Description

### IPGs and IPG Analogues

Studies have shown that A-type mediators modulate the activity of a number of insulin-dependent enzymes such as cAMP dependent protein kinase (inhibits), adenylate cyclase (inhibits) and cAMP phospho-diesterases (stimulates). In contrast, P-type mediators modulate the activity of insulin-dependent enzymes such as pyruvate dehydrogenase phosphatase (stimulates), glycogen synthase phosphatase (stimulates) and cAMP dependent protein kinase (inhibits). The A-type mediators mimic the lipogenic activity of insulin on adipocytes, whereas the P-type mediators mimic the glycogenic activity of insulin on muscle. Both A and P-type mediators are mitogenic when added to fibroblasts in serum free media. The ability of the mediators to stimulate fibroblast proliferation is enhanced if the cells are transfected with the EGF-receptor. A-type mediators can stimulate cell proliferation in the chick cochleovestibular ganglia (CVG).

Soluble IPG fractions having A-type and P-type activity have been obtained from a variety of animal tissues including rat tissues (liver, kidney, muscle brain, adipose, heart) and bovine liver. A and P-type IPG biological activity has also been detected in human liver and placenta, malaria parasitized RBC and mycobacteria. The ability of a polyclonal anti-inositolglycan antibody to inhibit insulin action on human placental cytotrophoblasts and BC3H1 myocytes or bovine-derived IPG action on rat diaphragm and CVG suggests cross-species conservation of many structural features. However, it is important to note that although the prior art includes these reports of A and P-type IPG activity in some biological fractions, the purification or characterisation of the agents responsible for the activity is not disclosed.

A-type substances are cyclitol-containing carbohydrates, also containing Zn²⁺ ion and optionally phosphate and having the properties of regulating lipogenic activity and inhibiting cAMP dependent protein kinase. They may also inhibit adenylate cyclase, be mitogenic when added to EGF-transfected fibroblasts in serum free medium, and stimulate lipogenesis in adipocytes.

P-type substances are cyclitol-containing carbohydrates, also containing Mn²⁺ and/or Zn²⁺ ions and optionally phosphate and having the properties of regulating glycogen metabolism and activating pyruvate dehydrogenase phosphatase. They may also stimulate the activity of glycogen synthase phosphatase, be mitogenic when added to fibroblasts in serum free medium, and stimulate pyruvate dehydrogenase phosphatase.

Methods for obtaining A-type and P-type IPGs are set out in detail in Caro et al, 1997, and in WO98/11116 and WO98/11117.

### Antagonists

As mentioned above, in the present invention, IPG antagonists include substances which have one or more of the following properties:
(a) Substances capable of inhibiting release of the IPGs from mast cells or basophils, e.g. in response to an allergen;
(b) Substances capable of reducing the level of IPGs released from mast cells, basophils or eosinophils by specifically binding to such IPGs; and/or,
(c) Substances capable of reducing the effects of IPGs released from mast cells, basophils or eosinophils, e.g. substances which compete with the mast cell/basophil/eosinophil derived IPGs, but which do not cause allergic stimulation of these cell types.

In one embodiment, the IPG antagonists can act to prevent IPG release in mast cells, basophils or eosinophils, e.g. in response to an allergen. An example of such an antagonist is an inhibitor of the enzyme GPI-PLD which is involved in the release of the IPG from the mast cell, basophil or eosinophil surface following allergen stimulation. Another inhibitor is an anti-GPI-PLD antibody which acts to prevent IPG release by inhibiting cleavage of the IPGs caused by the enzyme GPI-PLD.

Alternatively, the IPG antagonist can be an antibody capable of specifically binding to IPGs, e.g. to reduce IPG levels in a patient. Preferably, the antibodies are capable of specifically binding to IPGs produced by mast cells, basophils or eosinophils. The use of neutralising antibodies is preferred, e.g. antibodies which are capable of neutralising the activity of IPGs causing histamine release from mast cells, basophils or eosinophils. Examples of monoclonal antibodies capable of specifically binding IPGs are the antibodies produced by hybridoma cell lines 2F7, 2D1 and 5H6 deposited at European Collection of Cell Cultures (ECACC) under accession numbers 98051201, 98031212 and 98030901. Protocols for making monoclonal and polyclonal anti-IPG antibodies are set out below.

As the action of IPGs is generally tissue specific, IPGs derived from other tissues can provide a source of competitive antagonists to IPGs released from mast cells, basophils or eosinophils. An example of this type of antagonist is the rat liver A-type IPG described in the examples below, which in combination with an adjunct (in this case Ca²⁺) was antagonised the hexosaminidase release from basophil cell line RBL 2H3. Synthetic chemical compounds can also act as IPG antagonists. P and A-type IPGs suitable for screening for use as competitive antagonists are described above. Synthetic IPG analogues include compound C3, 1D-6-O-(2-amino-2-deoxy-α-D-glucopyranosyl)-myo-inositol 1,2-(cyclic phosphate), as prepared in Zapata et al, 1994, and compound C4, 1D-6-O-(2-amino-2-deoxy-α-D-glucopyranosyl)-chiro-inositol 1-phosphate, as prepared in Jaramillo et al, 1994.

In one embodiment, antibodies are useful IPG antagonists that can be used in the present invention. Protocols for obtaining anti-IPG antibodies are set out below. However, the deposited antibodies or other antibodies made using the protocols below can be subjected to the techniques of recombinant DNA technology to produce other antibodies or chimeric molecules which retain the specificity of the original antibody. Such techniques may involve introducing DNA encoding the immunoglobulin variable region, or the complementarity determining regions (CDRs), of an antibody to the constant regions, or constant regions plus framework regions, of a different immunoglobulin. See, for instance, EP 0184187 A, GB-A-2188638 or EP 0239400 A. A hybridoma producing a monoclonal antibody may be subject to genetic mutation or other changes, which may or may not alter the binding specificity of antibodies produced.

Antibodies may be obtained using techniques which are standard in the art. Methods of producing antibodies include immunising a mammal (e.g. mouse, rat, rabbit, horse, goat, sheep or monkey) with the protein or a fragment thereof. Antibodies may be obtained from immunised animals using any of a variety of techniques known in the art, and screened, preferably using binding of antibody to antigen of interest. For instance, Western blotting techniques or immunoprecipitation may be used (Armitage et al, Nature, 357:80-82, 1992). Isolation of antibodies and/or antibody-producing cells from an animal may be accompanied by a step of sacrificing the animal.

As an alternative or supplement to immunising a mammal with a peptide, an antibody specific for a protein may be obtained from a recombinantly produced library of expressed immunoglobulin variable domains, e.g. using lambda bacteriophage or filamentous bacteriophage which display functional immunoglobulin binding domains on their surfaces; for instance see WO92/01047. The library may be naive, that is constructed from sequences obtained from an organism which has not been immunised with any of the proteins (or fragments), or may be one constructed using sequences obtained from an organism which has been exposed to the antigen of interest.

Antibodies according to the present invention may be modified in a number of ways. Indeed the term "antibody" should be construed as covering any binding substance having a binding domain with the required specificity. Thus the invention covers antibody fragments, derivatives, functional equivalents and homologues of antibodies, including synthetic molecules and molecules whose shape mimics that of an antibody enabling it to bind an antigen or epitope.

Example antibody fragments, capable of binding an antigen or other binding partner are the Fab fragment consisting of the VL, VH, Cl and CH1 domains; the Fd fragment consisting of the VH and CH1 domains; the Fv fragment consisting of the VL and VH domains of a single arm of an antibody; the dAb fragment which consists of a VH domain; isolated CDR regions and F(ab')₂ fragments, a bivalent fragment including two Fab fragments linked by a disulphide bridge at the hinge region. Single chain Fv fragments are also included.

Humanised antibodies in which CDRs from a non-human source are grafted onto human framework regions, typically with the alteration of some of the framework amino acid residues, to provide antibodies which are less immunogenic than the parent non-human antibodies, are also included within the present invention

A hybridoma producing a monoclonal antibody according to the present invention may be subject to genetic mutation or other changes. It will further be understood by those skilled in the art that a monoclonal antibody can be subjected to the techniques of recombinant DNA technology to produce other antibodies or chimeric molecules which retain the specificity of the original antibody. Such techniques may involve introducing DNA encoding the immunoglobulin variable region, or the complementarity determining regions (CDRs), of an antibody to the constant regions, or constant regions plus framework regions, of a different immunoglobulin. See, for instance, EP 0184187 A, GB-A-2188638 or EP 0239400 A. Cloning and expression of chimeric antibodies are described in EP 0120694 A and EP 0125023 A.

Hybridomas capable of producing antibody with desired binding characteristics are within the scope of the present invention, as are host cells, eukaryotic or prokaryotic, containing nucleic acid encoding antibodies (including antibody fragments) and capable of their expression. The invention also provides methods of production of the antibodies including growing a cell capable of producing the antibody under conditions in which the antibody is produced, and preferably secreted.

### Pharmaceutical Compositions

The IPGs and IPG antagonists of the invention can be formulated in pharmaceutical compositions. These compositions may comprise, in addition to one or more of the mediators or antagonists, a pharmaceutically acceptable excipient, carrier, buffer, stabiliser or other materials well known to those skilled in the art. Such materials should be non-toxic and should not interfere with the efficacy of the active ingredient. The precise nature of the carrier or other material may depend on the route of administration, e.g. oral, intravenous, cutaneous or subcutaneous, nasal, intramuscular, intraperitoneal routes.

Pharmaceutical compositions for oral administration may be in tablet, capsule, powder or liquid form. A tablet may include a solid carrier such as gelatin or an adjuvant. Liquid pharmaceutical compositions generally include a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as sodium chloride injection, Ringer's injection, lactated Ringer's injection. Preservatives, stabilisers, buffers, antioxidants and/or other additives may be included, as required.

Whether it is a polypeptide, antibody, peptide, small molecule or other pharmaceutically useful compound according to the present invention that is to be given to an individual, administration is preferably in a "prophylactically effective amount" or a "therapeutically effective amount" (as the case may be, although prophylaxis may be considered therapy), this being sufficient to show benefit to the individual. The actual amount administered, and rate and time-course of administration, will depend on the nature and severity of what is being treated. Prescription of treatment, e.g. decisions on dosage etc, is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in Remington's Pharmaceutical Sciences, 16th edition, Oslo, A. (ed), 1980.

A composition may be administered alone or in combination with other treatments, either simultaneously or sequentially dependent upon the condition to be treated. Many of the conditions that can be treated using the invention are base on reaction to allergens. Thus, the treatments proposed herein could be combined with known treatments for these conditions.

### Materials and Methods

### Production of Polyclonal and Monoclonal Antibodies Against Inositolphosphoglycans (IPGs)

Inositolphosphoglycan (soluble form) obtained by PI-PLC treatment of GPI purified from rat liver by sequential Thin Layer Chromatography (TLC) was used to immunize New Zealand rabbits and Balb/c mice as described below. Alternatively, human IPGs or those from other species can be obtained using the methods described in Caro et al, 1997.

### Rabbit Immunisation Procedure

Two New Zealand rabbits were anaesthetized and then immunised with 750µg of IPG (soluble form) mixed in 1ml of PBS with 1ml of complete Freund's adjuvant (CFA). The antigen-adjuvant emulsion was administered 1.5ml by intradermal (id) injection and 0.5ml by intramuscular (im) injection. After one month, this protocol was repeated except that incomplete Freund's adjuvant (IFA) was used, and 1.5ml by administered by subcutaneous (sc) injection and 0.5ml by intramuscular (im) injection. This was repeated again on days 60, 90, 120 and 150.

### Mouse Immunisation Procedure

Four female Balb/c 6 weeks old mice were immunised with 60µg of IPG (soluble form) in 250µl of PBS with 250µl of CFA. The antigen-adjuvant emulsion was injected by intraperitoneal (ip) injection. After 21 days, the injection was repeated except that IFA was used. On days 42 and 63 all the animals were injected ip with IFA. On day 84, the best responder was injected 100µl PBS containing 60µg of IPG intravenous (iv) and 100µl PBS containing 60µg of IPG (ip). After 87 days, splenocytes from best responder were fused to myeloma cells using conventional techniques. Monitor test bleeds were realized regularly.

### Assay Procedures

### Histamine Assay

The extraction of HNMT was performed as discussed in Verburg et al, 1983, using fresh male Wistar rat kidney tissue. The tissue was homogenised with 0.25M sucrose solution (1:3 v/w) and centrifuged at 40,000G for 15 minutes. The supernatant was collected and adjusted to pH5.0 using 2M acetic acid solution. A second centrifugation was performed, at 40,000G for 10 minutes and the supernatant was adjusted to pH7.0 using 1M ammonium hydroxide. Solid ammonium hydroxide was added (0.54g per ml) to give an 82% saturated solution, which was stirred gently for 30 minutes before centrifugation at 40,000G for 15 minutes. The precipitate was resuspended in cold 25mM potassium phosphate buffer containing 0.1mM EDTA (pH7.5) (0.44ml buffer per mg original tissue). Overnight dialysis was performed against the resuspension buffer and the enzyme product was stored at -20°C until required. Histamine assays were performed to analyse the enzyme activity of the product.

The histamine detection assay is based on the transfer (methylation) of the 3H group from [³H] S-adenosyl-methionine ([³H]SAME) to histamine by the HNMT enzyme, to form tritiated N-π-methylhistamine, Verburg et al, 1983. The histamine detection assay was performed as outlined in Gitomer and Tipton, 1986, using 0.1µCi of [³H] S-adenosyl-methionine, 2mM S-adenosyl-methionine in a total reaction volume of 100µl. The reaction was initiated by the addition of enzyme and incubated at 37°C for 30 minutes, and terminated by the addition of 7% sodium borate solution, pH10.6. The tritiated histamine was extracted using toluene:isoamyl alcohol (1:1 v/v) and 800µl of the aqueous phase was added to 2ml Ultima Gold. The radioactivity was detected by scintillography.

Column separation was effected as discussed in Verburg et al, 1983, using potassium phosphate buffers containing 0.1mM EDTA (pH7.5). DEAE Cellulose separation was performed using 100mM and 750mM buffers, followed by separation over phenyl Sepharose, with elution using 400mM and 10mM buffers. All chromatographic procedures were performed using gas purged buffers, to reduce oxidative inactivation of the enzyme, and the fractions were maintained on ice. The protein content of the eluted fractions was ascertained by spectrophotometry at 280nm wavelength and only those fractions with the highest value were retained for study.

Accurate protein concentrations were ascertained by use of the Coomassie Plus protein assay reagent system (SIGMA), and comparison with BSA standards.

### N-acetyl-β-glucosaminidase assay

This assay was performed as described in Yasuda et al, 1995. An aliquot of cell supernatant was added to 10mM p-nitrophenyl-acetyl-β-D-glucosamine, maintained in 0.1M citrate buffer at pH4.0. The samples were incubated at 37°C for 30 minutes and the reaction terminated by the addition of 0.4M glycine buffer, pH10.6. Colour generated wad detected by spectrophotometry at 405nm wavelength on an ELISA reader.

### IL-4 assay

IL-4 was detected using the DuoSet IL-4 Detection Kit (Genzyme Diagnostics). The detection method was performed according to manufacturer's instructions. All suggested volumes were halved to maximise economic use of the reagents provided.

### Cell Experiments

### RBL-2H3 Cell Culture

The RBL-2H3 cell line was cultured so that the detection assays could be optimised without the need to withdraw human blood. The cells were cultured according to Gilfillan et al, 1992. Briefly, the cells were maintained in Eagles Modified Essential Medium supplemented with 10% heat inactivated FCS, 100 units /ml penicillin, 100µg/ml streptomycin/gentacmycin and 2mM L-glutamine at 37°C in a humidified atmosphere of 95% air and 5% carbon dioxide. Cell splitting was achieved using cell scrapers.

### Cell Lysis

Prior to performing experiments to determine the variation in mediator release following activation, it was necessary to determine the total concentration of each mediator per cell. This enabled mediator release to be expressed as a percentage of the cell's total capacity. These determinations were achieved by cell lysis. Three methods of lysis were optimised:
(1) Triton lysis: incubation with 1-10% Triton X-100 for 15 to 90 minutes.
(2) Heat lysis: incubation at 85°C for 2 to 15 minutes.
(3) Freeze lysis: incubation at -20°C for 30 minutes.

The efficiency of lysis was determined by microscopy and the mediators detected as described.

### IgE-Cross Linking

The IgE cross-linking was performed as described by Gilfillan et al, 1992. RBL-2H3 cells were grown to confluency and then replated in a 6-well plate at a density of 1x10⁶ cells per well. The cells were cultured overnight in supplemented medium containing anti-DNP IgE, washed twice with medium, and once with HEPES buffer (137mM sodium chloride, 2.7mM potassium chloride, 0.4mM disodium phosphate, 5.6mM glucose, 10mM HEPES, 1.8mM calcium chloride, 1.3mM magnesium sulphate, pH7.4). Mediator release was triggered by the addition of DNP-HSA (10ng/ml) diluted in HEPES buffer and incubation effected for 30 minutes. Mediator release was detected from the supernatant, as discussed in section 2.

Experiments were performed to ascertain the concentration of anti-DNP IgE antibody and DNP-HSA to achieve optimal mediator release.

### Elucidation of IgE status of Human Basophils:

### Basophil Extraction

A crude basophil extraction, based on dextran sedimentation, was performed as described in Pruzansky and Patterson, 1981. Briefly, blood was obtained by venipuncture and EDTA (pH7.5) was added to a final concentration of 25mM. Dextran-70 was added to a final concentration of 6% and the mixture was left to sediment. The upper, leukocyte containing, layer was removed and centrifuged at 200g for 12 minutes. The cell pellet was washed with HEPES buffered saline (140mM sodium chloride and 10mM HEPES, pH7.4) and resuspended in HEPES AGM (25mM HEPES, 110mM sodium chloride, 5 mM potassium chloride, 2mM calcium chloride, 1mM magnesium chloride and 0.3 mg/ml human serum albumin, pH7.4).

### Alcian Blue Staining

Alcian blue staining was performed as discussed in Gilbert and Ornstein, 1975. In summary, 100µl of cell suspension was diluted with 400µl of 0.1% EDTA in saline, 450µl staining solution (0.076% cetyl pyridinium chloride, 0.7% lanthanum chloride.6H₂O, 0.9% sodium chloride, 0.21% Tween 20, 0.143% alcian blue 8 GN in deionized water, filtered through a 1µ filter) and 50µl of 1M hydrochloric acid. After gentle agitation, the cells were counted in a Fuchs-Rosenthal Haemocytometer.

### Lactic Acid Stripping

Standardisation of experiments necessitated the use of the same basophils in each case. Therefore it was necessary to remove their native IgE and adsorb alternative IgE onto the surface. Surface bound IgE was dissociated from basophils with the use of the lactic acid elution method described by Sampson et al, 1989. The leukocytes were pelleted and resuspended in 5ml lactic acid solution (0.01M lactic acid, 110mM sodium chloride, 5mM potassium chloride, pH3.9). The suspension was incubated at 23°C for three and a half minutes, after which time the lactic acid was diluted with 30ml PIPES buffer (25mM PIPES, 110mM sodium chloride, 5mM potassium chloride, 0.03% human serum albumin, 0.1%D-glucose, pH7.4).

### Passive Sensitisation

Passive sensitisation method was based on that described in Pruzanksy et al, 1981 and Levy and Osler, 1966. Cells were incubated at with 250ng IgE per million basophils 37°C for 60 minutes.

### HRF assay

A 1 in 10 serial dilution of HRF was effected prior to incubation at 37°C for 45 minutes in a heating block. The mediator chosen for study was β-hexosaminidase.

### Alkaline Inactivation of Foetal Calf Serum

Inactivation of GPI-PLD activity in Foetal Bovine Serum was achieved according to the method of Kung et al (Biochimica et Biophysica Acta, 1997, 1357, 329 - 338). Briefly, FCS was adjusted to pH 11 using concentrated hydrochloric acid, and incubated for 1 hour at 37°C. After this time, the pH was adjusted to 7.4, and GPI-PLD activity was determined using an enzymatic assay (Davitz et al, 1989).

In order to ascertain how this serum affected the function of RBL-2H3 cells, the FBS in the culture medium was replaced with the inactive serum, and the cells were cultured as normal.

### IgE Cross Linking Assay

RBL-2H3 cells were grown to confluence, after which time the adherent cells were removed from the culture flask using a cell scraper. The cell density was determined, using a haemocytometer, and adjusted to 2 x 10⁵ per ml. The cells were seeded at 1ml per well in a 24 well culture plate and cultured for overnight at 37°C in a humidifed 5% CO₂ incubator.

The overnight culture media was aspirated and replaced with fresh media containing Rat IgE anti-DNP at 3µg/ml. After a 2 hour incubation period, the media was aspirated, and the cells were washed twice, with HEPES Buffered Saline. Cross-linking was achieved by the addition of 200µls of DNP-Albumin at 100ngs/ml (diluted in HEPES AGM), and incubation for 2 hours. The release of the total mediator content of the cells was accomplished by incubation with 200µls 5% Triton X-100 detergent. In addition, the cells were stimulated to release using the calcium ionophore, A23187.

Mediator release was determined using a colorimetric assay to detect the presence of β-hexosaminidase and compared with the total cell β-hexosaminidase content (Yasuda et al, Int. Immunol., 1995).

### Results

### IPGs obtained from basophil cell line RBL 2H3 cause the release of histamine from the RBL 2H3 cells

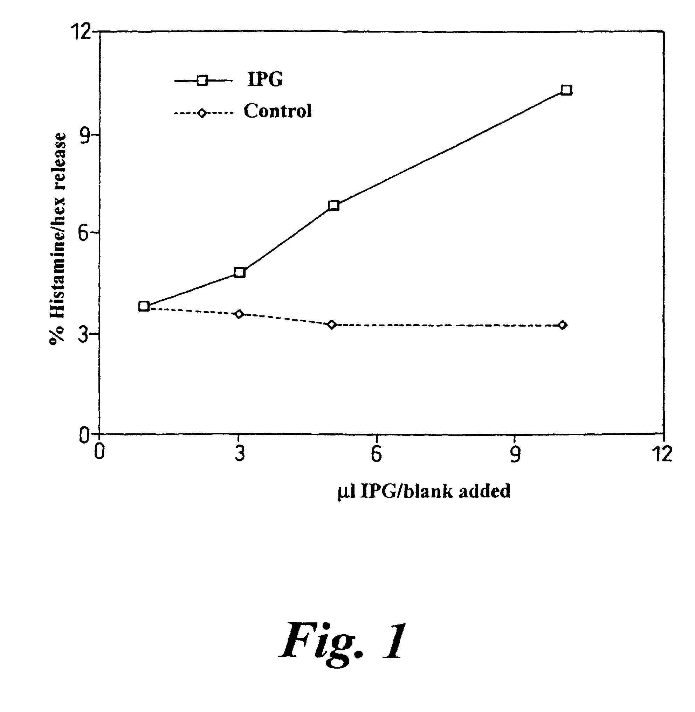

Figure 1 shows that IPGs obtained from a basophil cell line RBL 2H3 cause the release of histamine from the RBL 2H3 cells. The basophil derived IPG was obtained by cross-linking the IgE receptors on the surface of the RBL 2H3 cells which stimulates the interaction of an allergen with the basophil. Specifically, RBL 2H3 cells were sensitised with anti-DNP IgE (which binds to the IgE receptors present on the basophil surface) and then triggered with the antigen DNP-HSA. This antigen (DNP-HSA) triggering results in histamine release. The culture supernatants were extracted and Figure 1 shows that:
(a) IPG could be obtained from the supernatants and that allergen stimulation results in IPG release.
(b) The purified IPGs when added to non-allergen stimulated cells resulted in the release of histamine indicating that the IPG was the second messenger for allergic stimulation.

### Histamine release caused by IPGs is tissue specific

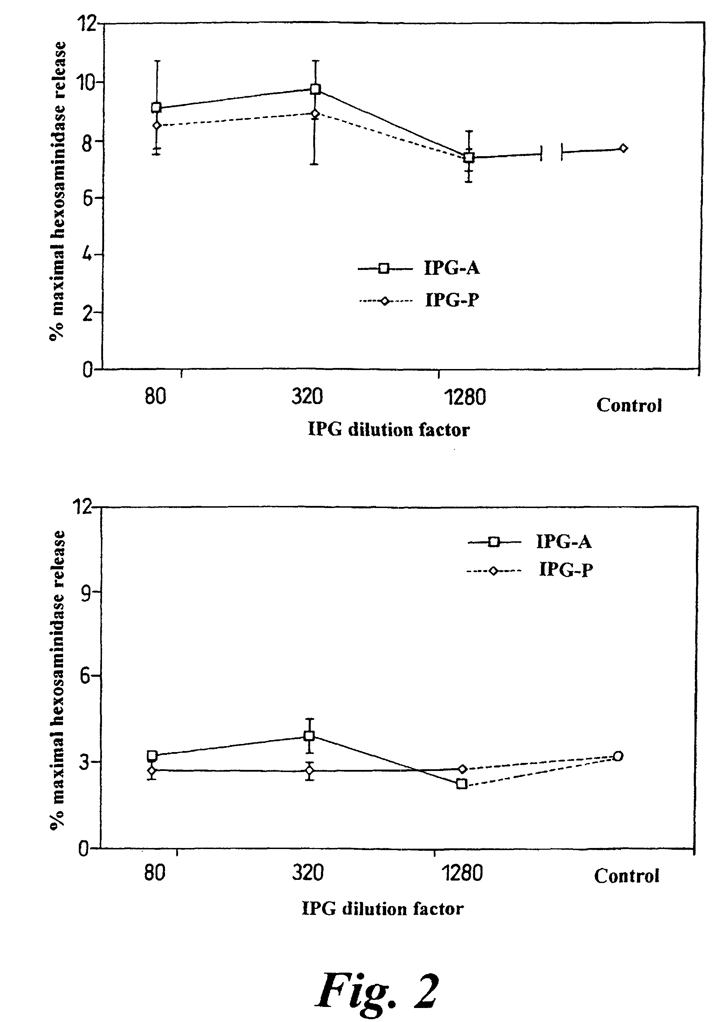

Figure 2 shows that the response is tissue-specific since IPGs isolated from human placental were not able to cause histamine release (the hexosaminidase release (y axis) is a surrogate marker for histamine release).

### Rat liver A-type IPG is a specific antagonist of histamine release

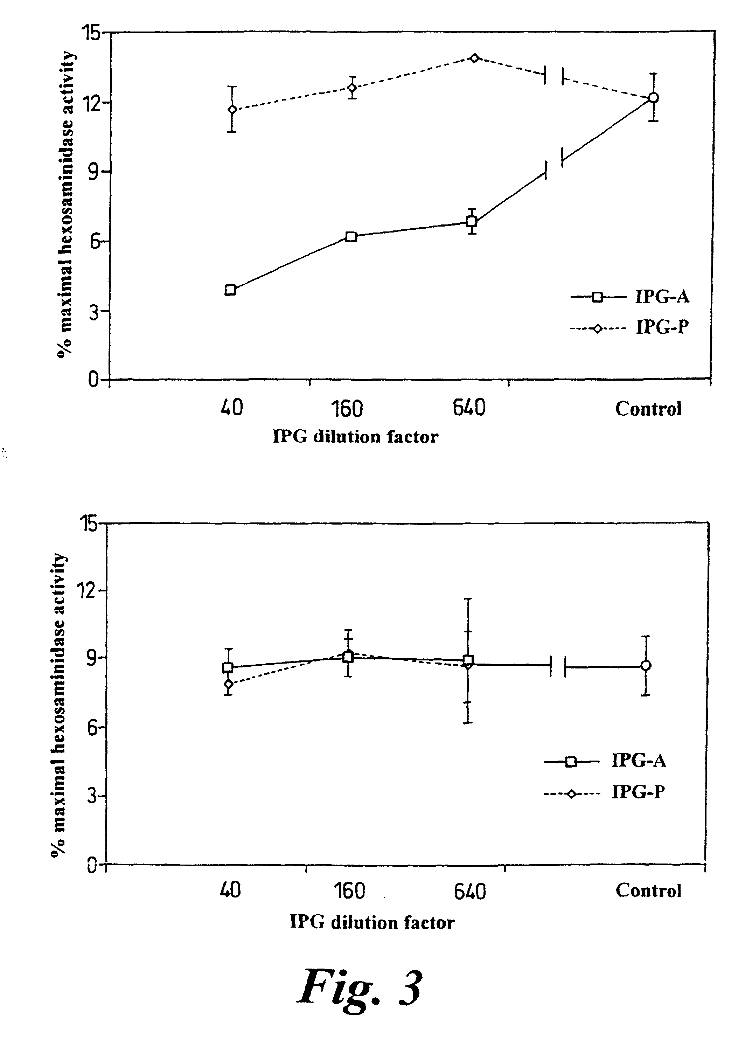

Figure 3 shows that rat liver P-type IPG has no effect on the RBL 2H3 cells. In contrast, the rat A-type liver IPG was able to block some of the spontaneous release. The effect is specific since no inhibition was seen in the absence of calcium (Figure 3 bottom).

### The role of GPI-PLD in type one hypersensitivity reactions

The role of Glycosylphosphatidyl-inositol Phospholipase D (GPI-PLD) in the Type One Hypersensitivity reaction was then examined. This reaction involves the cross-linking of IgE receptors on the mast cell surface, leading to the release of allergic mediators, and can be experimentally reproduced using rat basophilic leukaemia cell line, RBL-2H3. These cells naturally have unoccupied IgE receptors (Fc∈R1, or high-affinity receptors), allowing them to be passively sensitised with an IgE isotype of choice. The allergic reaction can be mimicked by cross-linking of the IgE.

Previous research indicates that RBL-2H3 cells derive their GPI-PLD from the culture serum (data not shown). Therefore, it follows that inactivation of this external source of GPI-PLD would deprive the cells of any further enzyme.

### Results indicate that the alkaline treatment of FBS

severely depleted the serum's GPI-PLD activity (data not shown).

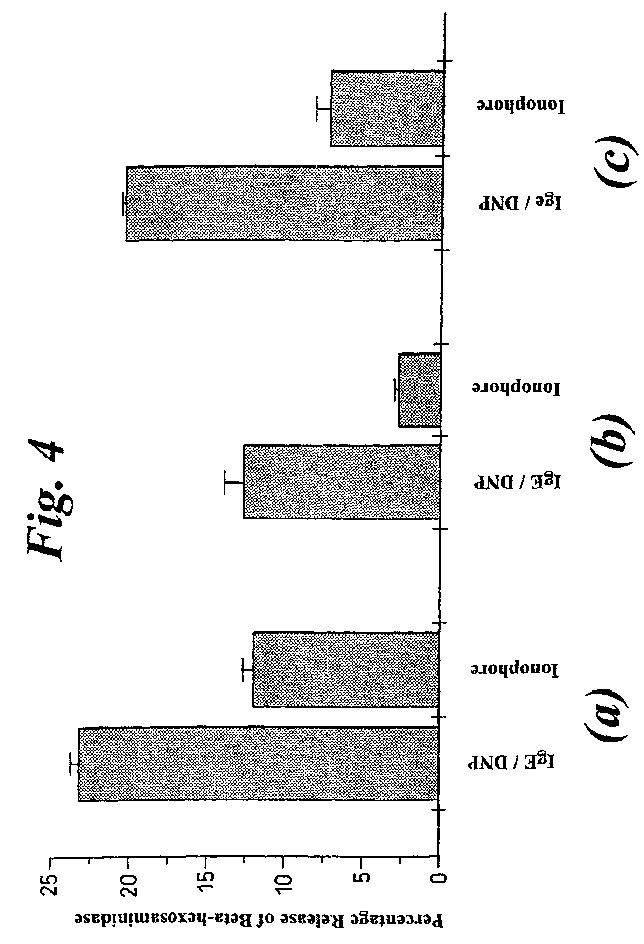

When the inactive serum was used in the culture medium, the appearance of the cells was not dramatically altered, although their adherence seemed to be slightly reduced. However, the cells rapidly lost their ability to release mediators, following cross linking of the FceR1 receptor on the cell's surface, or following stimulation with A23187 (Figure 4b). The loss of activity was approximately half, when compared with the release from cells which were cultured normally (Figure 4a) .

In order to ascertain whether these "inactive" cells had been irreparably damaged by their incubation in the alkaline treated FBS, the cells were transferred back into medium in which the FBS had not been altered. When the cells are split at a low density, allowed to gain confluence, and then used in the stimulation experiments, the cells partially regained their capacity to respond to both Fc∈R1 cross linking and A23187 stimulation (Figure 4c). This indicates that the cells have not been irreparably damaged by their altered culture conditions.

Alkaline treatment of FBS appears to affect the function of a serum component, which is essential in the release of mediators from RBL-2H3 cells, following stimulation using two methods.

Although assay of GPI-PLD indicated a loss of activity of the enzyme, and this enzyme has been suggested as having an important function in basophil function, it is highly probable that there are other components of the serum, which would be affected by alkaline conditions. It is widely known, for example, that the growth factor TGF-β would be activated by this treatment.

The results suggesting that the stimulation of basophils by ionophore is affected by this serum component may also involve GPI-PLD depletion in this process. We have found the following: GPI-PLD depletion results in the down expression of caveolin, an important component of caveolae; calcium uptake and ionophore action is known to occur in caveolae.

### Deposits

The deposit of hybridomas 2F7, 2D1 and 5H6 in support of this application was made at the European Collection of Cell Cultures (ECACC) under the Budapest Treaty by Rademacher Group Limited (RGL) (formerly Hoeft Rademacher Limited), The Windeyer Building, 46 Cleveland Street, London W1P 6DB, UK. The deposits have been accorded accession numbers accession numbers 98051201, 98031212 and 98030901. RGL authorises the University College London to refer to the deposited biological materials in this application and both RGL and University College London give their unreserved and irrevocable consent to the materials being made available to the public in accordance with appropriate national laws governing the deposit of these materials, such as Rules 28 and 28a EPC. The expert solution under Rule 28(4) EPC is also hereby requested.

### References:

The references mentioned herein are incorporated by reference in their entirety.
WO98/11116 and WO98/11117 (Rademacher Group Limited).
Rademacher et al, Brazilian J. Med. Biol. Res., 27:327-341, 1994.
Caro et al, Biochem. Molec. Med., 61:214-228, 1997.
Kunjara et al, In: Biopolymers and Bioproducts: Structure, Function and Applications, Ed Svati et al, 301-305, 1995.
Sutton and Gould, Nature, 366:421-428, 1993.
Sampson et al, New England Journal of Medicine, 321(4):228-232, 1989.
Kuna et al, J. Immunol., 150(5):1932-1943, 1993.
Solley et al, J. Clin. Invest., 58(2):408, 1976.
MacLean et al, Clin. Invest., 1:63, 1971.
Lichtenstein, J. Allergy Clin. Immunol., 91(5:1): 814-820, 1988.
Liu et al, J. Immunol., 136:2588, 1986.
MacDonald et al, Science 269:688-690, 1995.
Sim et al, J. Allergy Clin. Immunol., 89(6):1157-1165, 1992.
Fisher et al, J. Allergy Clin. Immunol., 79:196, 1987.
Verburg et al, Life Sciences, 32:2855-2867, 1983.
Gitomer and Tipton, Biochem. J., 233:669-676, 1986.
Yasuda et al, Int. Immunonology, 7(2):251-8, 1995.
Gilfillan et al, J. Immunol., 149(7):2445-2451, 1992.
Zapata et al, Carbohydrate Res., 264;21-31, 1994.
Jaramillo et al, J. Org. Chem, 59:3135-3141, 1994.
Davitz et al, J. Biol. Chem., 264:13760-13764, 1989.
Yasuda et al, Int. Immunol., 7:251-258, 1995.

## Claims

1. Use of an antagonist in the preparation of a medicament for the treatment of conditions mediated by the release of IPGs from mast cells, basophils or eosinophils, wherein the antagonist is:
(a) a substance which is capable of inhibiting release of the IPGs by inhibiting the enzyme GPI-PLD;
(b) a substance which is capable of specifically binding to the IPGs and inhibiting the release of histamine caused by the IPGs; or
(c) a substance which is capable of competing with IPGs released from mast cells, basophils or eosinophils but which does not cause allergic stimulation of these cell types.

2. The use of claim 1, wherein the condition mediated by release of IPGs is atopic dermatitis, food hypersensitivity, allergies including seasonal, contact, drug, pollen, insect allergies, asthma (early and late phase), allergic interstitial pneumonitis, eczema, environmental lung disease, or another disorders mediated by infiltration of mast cells, basophils or eosinophils, or cells within their respective lineages.

3. The use of claim 1 or claim 2, wherein the IPG antagonist is an anti-IPG antibody.

4. The use of claim 1 or claim 2, wherein the IPG antagonist is a substance capable of inhibiting or preventing IPG release in mast cells, basophils or eosinophils in response to an allergen.

5. The use of claim 4, wherein the antagonist is an inhibitor of the enzyme GPI-PLD.

6. The use of claim 5, wherein the antagonist is an antibody capable of inhibiting IPG release by inhibiting cleavage of the IPGs caused by the enzyme GPI-PLD.

7. The use of claim 1 or claim 2, wherein the IPG antagonist is a competitive antagonists of the IPGs released from mast cells, basophils or eosinophils.

8. The use of claim 7, wherein when the medicament is used to treat a human patient, the competitive IPG antagonist is an IPG derived from a non-human species.

9. The use of claim 8, wherein the antagonist is A-type IPG as obtainable from rat liver.

10. An inositolphosphoglycan (IPG) as obtainable from mast cells, basophils or eosinophils which is capable of causing histamine release from mast cells, basophils or eosinophils.

11. An inositolphosphoglycan (IPG) as obtainable from mast cells, basophils or eosinophils which is capable of causing histamine release from mast cells, basophils or eosinophils for use in a method of screening for antagonists of said IPG.

## Patentansprüche

1. Verwendung eines Antagonisten bei der Herstellung eines Medikaments zur Behandlung von Leiden, die durch die Freisetzung von IPGs aus Mastzellen, Basophilen oder Eosinophilen vermittelt werden, worin der Antagonist
(a) eine Substanz ist, die fähig ist, die Freisetzung der IPGs durch Hemmung des Enzyms GPI-PLD zu hemmen;
(b) eine Substanz ist, die fähig ist, sich spezifisch an die IPGs zu binden und die durch die IPGs verursachte Freisetzung von Histamin zu hemmen; oder
(c) eine Substanz ist, die fähig ist, mit von Mastzellen, Basophilen oder Eosinophilen freigesetzten IPGs zu konkurrieren, aber keine allergische Stimulierung dieser Zelltypen hervorruft.

2. Verwendung nach Anspruch 1, worin das durch Freisetzung von IPGs vermittelte Leiden atopische Dermatitis, Nahrungsmittelüberempfindlichkeit, eine Allergie, einschließlich saisonaler, Kontakt-, Arzneimittel-, Pollen-, Insekten-Allergien, Asthma (frühe und späte Phase), allergische interstitiale Pneumonitis, ein Ekzem, eine umgebungsbedingte Lungenerkrankung oder eine andere Störung ist, die durch Infiltration von Mastzellen, Basophilen oder Eosinophilen oder Zellen innerhalb ihrer jeweiligen Linien vermittelt wird.

3. Verwendung nach Anspruch 1 oder 2, worin der IPG-Antagonist ein Anti-IPG-Antikörper ist.

4. Verwendung nach Anspruch 1 oder 2, worin der IPG-Antagonist eine Substanz ist, die fähig ist, die IPG-Freisetzung in Mastzellen, Basophilen oder Eosinophilen als Reaktion auf ein Allergen zu hemmen oder zu verhindern.

5. Verwendung nach Anspruch 4, worin der Antagonist ein Inhibitor des Enzyms GPI-PLD ist.

6. Verwendung nach Anspruch 5, worin der Antagonist ein Antikörper ist, der fähig ist, die IGP-Freisetzung zu hemmen, indem er die durch das Enzym GPI-PLD verursachte Spaltung der IPGs hemmt.

7. Verwendung nach Anspruch 1 oder 2, worin der IPG-Antagonist ein kompetitiver Antagonist der von Mastzellen, Basophilen oder Eosinophilen freigesetzten IPGs ist.

8. Verwendung nach Anspruch 7, worin, wenn das Medikament verwendet wird, um einen Menschen zu behandeln, der kompetitive IPG-Antagonist ein IPG ist, das von einer anderen Spezies als dem Menschen stammt.

9. Verwendung nach Anspruch 8, worin der Antagonist aus Rattenleber erhältliches A-Typ-IPG ist.

10. Inositolphosphoglykan (IPG), das aus Mastzellen, Basophilen oder Eosinophilen erhältlich ist und fähig ist, die Histamin-Freisetzung aus Mastzellen, Basophilen oder Eosinophilen zu bewirken.

11. Inositolphosphoglykan (IPG), das aus Mastzvellen, Basophilen oder Eosinophilen erhältlich ist und fähig ist, die Histamin-Freisetzung aus Mastzellen, Basophilen oder Eosinophilen zu bewirken, zur Verwendung in einem Verfahren zum Screenen auf Antagonisten des IPG.

## Revendications

1. Utilisation d'un antagoniste dans la préparation d'un médicament pour le traitement des conditions provoquées par la libération des IPG par les mastocytes, les basophiles ou les éosinophiles, où l'antagoniste est :
(a) une substance qui est capable d'inhiber la libération des IPG par inhibition de l'enzyme GPI-PLD ;
(b) une substance qui est capable de se lier spécifiquement aux IPG et d'inhiber la libération de l'histamine provoquée par les IPG ; et
(c) une substance qui est capable de rentrer en compétition avec les IPG libérés par les mastocytes, les basophiles ou les éosinophiles mais qui ne provoque pas une stimulation allergique de ces types de cellules.

2. Utilisation de la revendication 1, où la condition provoquée par la libération des IPG est la dermatite atopique, l'hypersensibilité aux aliments, des allergies comprenant saisonnière, de contact, aux médicaments, au pollen, des allergies dues aux insectes, l'asthme (phase précoce et tardive) une pneumonite interstitielle allergique, l'eczéma, une maladie des poumons due à l'environnement ou d'autres troubles provoqués par une infiltration des mastocytes, des basophiles ou des éosinophiles ou des cellules dans leurs lignées respectives.

3. Utilisation de la revendication 1 ou de la revendication 2, où l'antagoniste des IPG est un anticorps anti-IPG.

4. Utilisation de la revendication 1 ou de la revendication 2, où l'antagoniste des IPG est une substance capable d'inhiber ou de prévenir la libération des IPG dans les mastocytes, les basophiles ou les éosinophiles en réponse à un allergène.

5. Utilisation de la revendication 4, où l'antagoniste est un inhibiteur de l'enzyme GPI-PLD.

6. Utilisation de la revendication 5, où l'antagoniste est un anticorps capable d'inhiber la libération des IPG par l'inhibition de la scission des IPG provoquée par l'enzyme GPI-PLD.

7. Utilisation de la revendication 1 ou de la revendication 2, où l'antagoniste des IPG est un antagoniste compétitif des IPG libérés par les mastocytes, les basophiles ou les éosinophiles.

8. Utilisation de la revendication 7, où, quand le médicament est utilisé pour traiter un patient humain, l'antagoniste des IPG compétitif est un IPG dérivé d'une espèce non humaine.

9. Utilisation de la revendication 8, où l'antagoniste est IPG du type A tel qu'il peut être obtenu du foie du rat.

10. Inositolphosphoglycane (IPG) tel qu'il peut être obtenu de mastocytes, de basophiles ou d'éosinophiles, qui est capable de provoquer une libération d'histamine par les mastocytes, les basophiles ou les éosinophiles.

11. Inositolphosphoglycane (IPG), tel qu'il peut être obtenu de mastocytes, de basophiles ou d'éosinophiles, qui est capable de provoquer une libération d'histamine par les mastocytes, les basophiles ou les éosinophiles pour une utilisation dans une méthode de dépistage des antagonistes dudit IPG.
